# EUROPEAN PATENT APPLICATION

(11) **EP 2 952 204 A1**
(43) Date of publication of application: **09.12.2015**
(21) Application number: 13873368.8
(22) Date of filing: 23.05.2013
(51) Int. Cl.: A61K 38/16, A61K 38/17, A61K 48/00, A61K 47/40

(54) **PHARMACEUTICAL COMPOSITION FOR REDUCING OR INHIBITING SCAR FORMATION, CONTAINING BMP-7 AND DILUTING AGENT**

(30) Priority: 01.02.2013 KR 20130011646
(71) Applicant: Eyegene Inc., Seoul 121-912 (KR)
(72) Inventor: CHO, Yang Je, Seoul 140-906 (KR); JANG, Jin Wook, Seoul 121-798 (KR); LIM, Hyeong Joon, Goyang-si Gyeonggi-do 410-361 (KR)
(74) Representative: Brevalex
(86) International application number: PCT/KR2013/004531
(87) International publication number: WO 2014/119823

(57) **Abstract**

The present invention relates to: a pharmaceutical composition for reducing or inhibiting scar formation, including bone morphogenetic protein 7 (BMP-7) and a specific diluting agent; and a method for reducing or inhibiting scar formation by using the same. The composition of the present invention can be effectively used as an eye drop and the like for preventing scar formation which can be caused by plastic surgery, corneal laser surgery and the like.

<110>    EYEGENE Inc.

<120>    Compositions for reducing or inhibiting the formation of scar
         comprising BMP-7 and excipients

<130>    PP130059

<150>    KR10-2013-0011646
<151>    2013-02-01

<160>    5

<170>    KopatentIn 2.0

<210>    1
<211>    24
<212>    DNA
<213>    Artificial Sequence

<220>
<223>    foward primer for collagen I


<400>    1
tcggcgagag catgaccgat ggat                                              24


<210>    2
<211>    24
<212>    DNA
<213>    Artificial Sequence

<220>
<223>    reverse primer for collagen I


<400>    2
gacgctgtag gtgaagcggc tgtt                                              24


<210>    3
<211>    19
<212>    DNA
<213>    Artificial Sequence

<220>
<223>    foward primer for GAPDH


<400>    3
gccaaagggt catcatctc                                                    19


<210>    4
<211>    20
<212>    DNA
<213>    Artificial Sequence

<220>
<223>    reverse primer for GAPDH


<400>    4

gtagaggcag ggatgatgtt                                                    20

<210>    5
<211>    139
<212>    PRT
<213>    Homo sapiens

<220>
<221>    PEPTIDE
<222>    (1)..(139)
<223>    BMP-7

<400>    5
Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu
    130                 135

## Description

### Technical Field

The present invention relates to a pharmaceutical composition for reducing or inhibiting scar formation, containing bone morphogenetic protein 7 (BMP-7) and a particular excipient.

### Background Art

Scars are formed as a part of the body's natural healing process in which the body generally initiates a complete and prompt wound healing reaction to reconstitute and restore tissue. However, in some cases, this normal healing procedure may cause an excessive scar tissue. The formation of excessive scar tissue is an important problem that affects surgical and healing results in some kinds of surgery or injury. For the eyeball, the scar formation after surgery may determine the success of the surgery. This is especially true in glaucoma, which is a disease that causes blindness. In this case, the results of glaucoma surgery have been improved using some scar formation preventing treatments, but these are restricted to clinical uses due to serious complications thereof. The other examples of excessive scar tissue formation that negatively affects the surgical results are adhesiolysis surgery, angioplasty, spinal surgery, vascular surgery, cardiac surgery, and the like.

Wound healing includes various steps. Various growth factors are secreted in wound areas during the wound healing procedure. Among the growth factors, transforming growth factor-β (TGF-β) is a critical mediator that regulates fibrosis and inflammation. TGF-β promotes transdifferentiation into myofibroblasts causing keratocyte proliferation and scar formation. Scars formed in the cornea during the wound healing procedure may give rise to vision loss. Therefore, agents that can effectively inhibit the activation of TGF-β may be promising candidates as a wound treatment.

Bone morphogenetic protein 7 (BMP-7) is a material involved in bone formation, and it is known that BMP-7 plays an important role in forming teeth and eyeballs during the ontogenetic procedure, but is not generated for adults (Dev Biol. 1999 Mar 1;207(1):176-88; Exp Cell Res. 1997 Jan 10;230(1):28-37). In addition, it has been reported that BMP-7 can prevent the progression of fibrosis and reduce the scar formation.

Throughout the entire specification, many papers and patent documents are referenced and their citations are represented. The disclosure of cited papers and patent documents are entirely incorporated by reference into the present specification, and the level of the technical field within which the present invention falls, and details of the present invention are explained more clearly.

### Detailed Description of the Invention

### Technical Problem

The present inventors have studied and endeavored to develop a safe excipient that can be applied to an eye solution (pharmaceutical composition) containing BMP-7. As a result, the present inventors have found that collagen, chitosan, and cyclodextrin do not exhibit cytotoxicity and can inhibit the formation of TGF-β-induced myofibroblasts when used together with BMP-7, and have completed the present invention.

Therefore, the present invention has been made in view of the above-mentioned problems, and an aspect of the present invention is to provide a pharmaceutical composition for reducing or inhibiting scar formation.

Another aspect of the present invention is to provide a method for reducing or inhibiting scar formation.

Other purposes and advantages of the present disclosure will become more obvious with the following detailed description of the invention, claims, and drawings.

### Technical Solution

In accordance with an aspect of the present invention, there is provided a pharmaceutical composition for reducing or inhibiting scar formation, the composition containing: (a) a pharmaceutically effective amount of bone morphogenetic protein 7 (BMP-7); and (b) at least one excipient selected from the group consisting of hyaluronic acid, chitosan, and cyclodextrin.

In accordance with another aspect of the present invention, there is provided a method for reducing or inhibiting scar formation, the method including administering to a subject, a therapeutically effective amount of a pharmaceutical composition containing at least one excipient selected from the group consisting of hyaluronic acid, chitosan, and cyclodextrin.

The present inventors have studied and endeavored to develop a safe excipient that can be applied to an eye solution (pharmaceutical composition) containing BMP-7. As a result, the present inventors have found that collagen, chitosan, and cyclodextrin do not exhibit cytotoxicity and can inhibit the formation of TGF-β-induced myofibroblasts when used together with BMP-7.

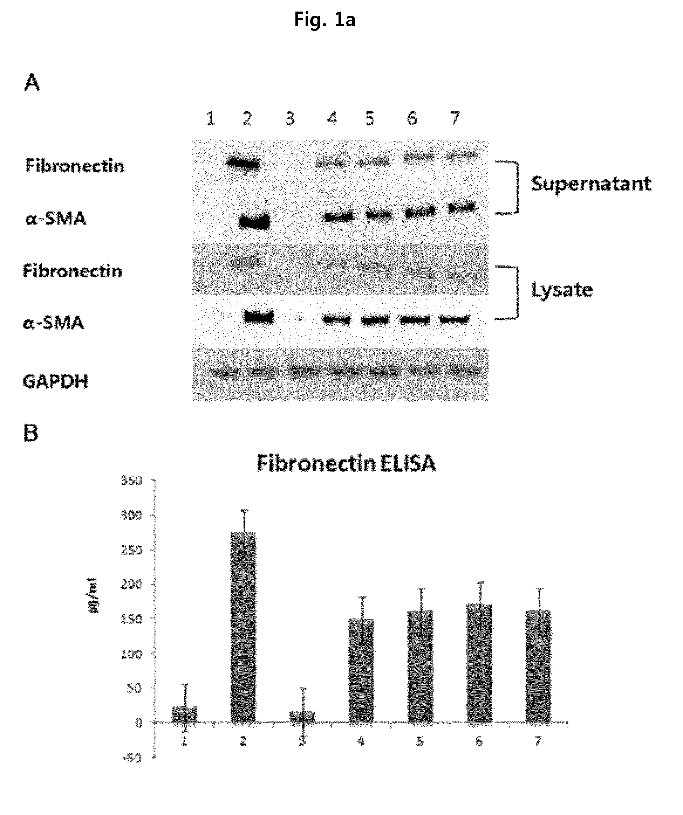

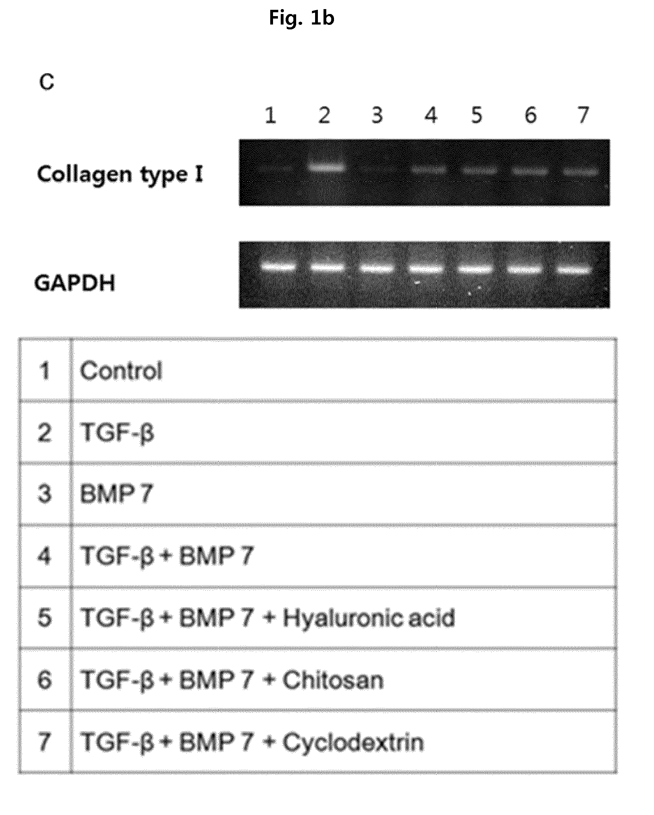

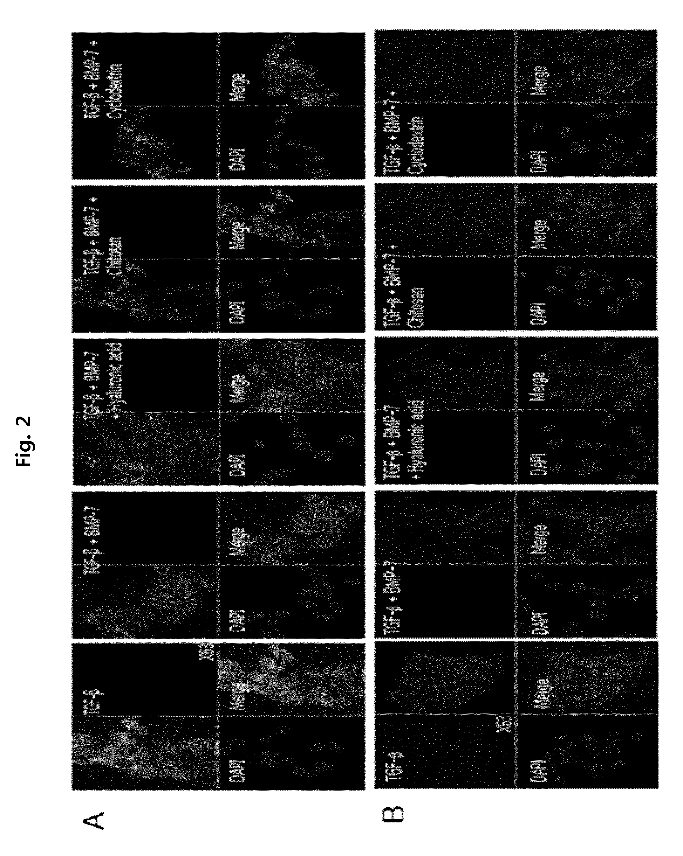

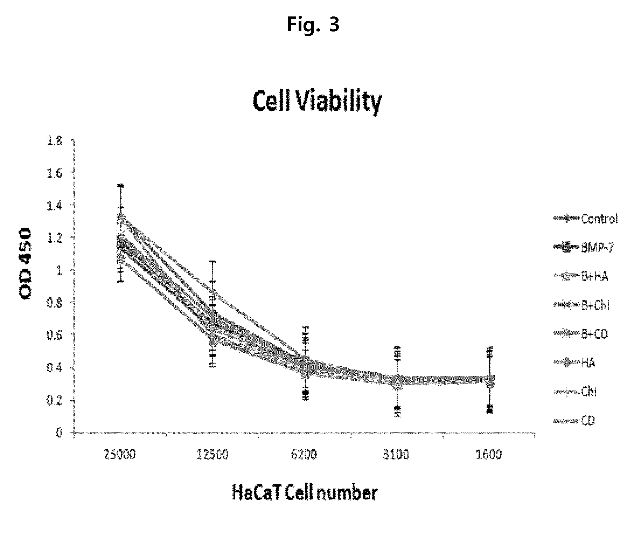

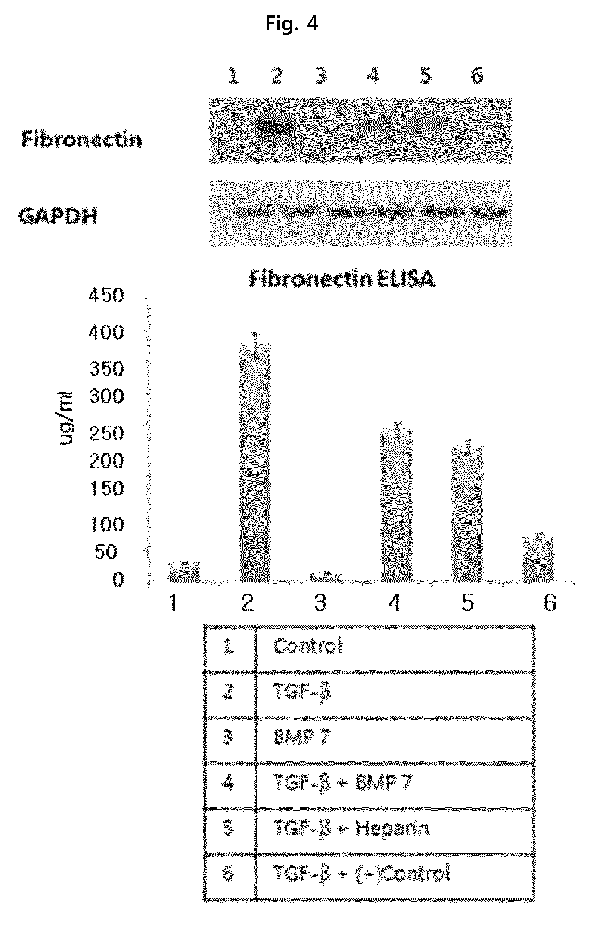

As proved in examples below, BMP-7 exhibited an equivalent level of scar formation inhibitory activity to heparin known as a scar formation inhibitor (see FIG. 4). In addition, as proved in examples below, hyaluronic acid, chitosan, and cyclodextran did not inhibit the scar formation inhibitory activity of BMP-7 (see FIGS. 1 and 2), and did not exhibit cytotoxicity (see FIG. 3).

As used herein, the term "scar" refers to a mark left on the skin by the healing of the injured skin. When even the deep layer of the dermal layer is damaged by surgery or external injury, collagen in the dermal layer that keeps the degree of tension of the skin hyperproliferates, and thus pushes through the thinned skin to leave a scar even after the wound is healed. This kind of scar is referred to as a general scar. The general scar is a result from scar healing, but this procedure abnormally occurs, resulting in a hypertrophic scar or keloid.

The composition of the present invention contains a pharmaceutically effective amount of BMP-7 that can reduce or inhibit scar formation at the time of healing scars. According to an embodiment of the present invention, the scar is a scar that occurs in the eyeball. In a particular embodiment, the scar formed in the eyeball is a scar formed on the surface of the eyeball, such as cornea or sclera.

The pharmaceutical composition of the present invention may contain the sequence of human BMP-7 and biological equivalents thereof within the range in which the scar formation inhibitory activity is exhibited. For example, in order to further improve scar formation inhibitory activity and/or other biological characteristics of BMP-7, the amino acid sequence of BMP-7 may be further modified. This modification includes, for example, residue deletion, insertion, and/or substitution with respect to the BMP-7 sequence.

According to an embodiment of the present invention, the human BMP-7 includes the amino acid sequence of SEQ ID NO: 5.

According to an embodiment of the present invention, the hyaluronic acid, chitosan, and cyclodextrin also include pharmaceutically acceptable salts and derivatives thereof. As used herein, the term "pharmaceutically acceptable salt(s)" refers to formulation (s) of a compound that does not cause significant irritation to an organism to which the composition is administered and does not abrogate the biological activity and properties of the compound. The pharmaceutically acceptable salt(s) is prepared by the general methods known in the art, using pharmaceutically acceptable, substantially nontoxic organic and inorganic acids. The acids include: inorganic acids, such as hydrochloric acid, bromic acid, sulfuric acid, nitric acid, and phosphoric acid; and organic acids, such as sulfonic acids including, for example, methane sulfonic acid, ethane sulfonic acid, and p-toluene sulfonic acid, tartaric acid, formic acid, citric acid, acetic acid, trichloroacetic acid, trifluoroacetic acid, capric acid, isobutyric acid, malonic acid, succinic acid, phthalic acid, gluconic acid, benzoic acid, lactic acid, fumaric acid, maleinic acid, and salicylic acid. In addition, the compound of the present invention may react with bases to form ammonium salts; alkali metal salts, such as sodium or potassium salt; alkali earth metal salts, such as calcium or magnesium salt; salts of organic bases, such as dicyclohexylamine, N-methyl-D-glucamine, and tris(hydroxymethyl) methylamine; and amino acid salts, such as arginine and lysine.

Examples of the pharmaceutically acceptable salt of the hyaluronic acid may include inorganic salts, such as hyaluronic acid sodium, hyaluronic acid magnesium, hyaluronic acid zinc, and hyaluronic acid cobalt, and organic salts, such as hyaluronic acid tetrabutyl ammonium.

Examples of the derivatives of the hyaluronic acid may include hyaluronic acid ester, amides of hyaluronic acid, deacetylated material of hyaluronic acid, and decarboxylated hyaluronic acid.

Examples of the chitosan derivative may include acetylated, alkylated, or sulfonated chitosan, and are specifically disclosed in Roberts, Chitin Chemistry, Macmillan, 1992, 166.

Examples of the cyclodextrin derivative may include hydroxypropyl cyclodextrin, carboxymethyl cyclodextrin, sulfobutyl cyclodextrin, amino cyclodextrin, dimethyl cyclodextrin, cyclodextrin phosphate, hydroxyethyl cyclodextrin, acetyl cyclodextrin, ethyl cyclodextrin, trimethyl cyclodextrin, carboxymethyl cyclodextrin, glucosyl cyclodextrin, 6-0-α-maltocyl cyclodextrin, butyl cyclodextrin, sulfate cyclodextrin, N,N-diethylaminoethyl cyclodextrin, tert-butylsilyl cyclodextrin, silyl[(6-O-tert-butyldimethyl)-2,3-di-O-acetyl)-cyclodextrin, succinyl-(2-hydroxypropyl)-cyclodextrin, succinyl cyclodextrin, sulfopropyl cyclodextrin, polycyclodextrin, and the like.

According to an embodiment of the present invention, the subject is a mammal including a human being.

The pharmaceutical composition of the present invention contains a pharmaceutically effective amount of BMP-7. As used herein, the term "pharmaceutically effective amount" refers to an amount of an active ingredient or a pharmaceutical composition that induces a biological or medical reaction in tissue systems, animals, or humans, and is considered by researchers, veterinarians, doctors, or other clinicians. The effective dosage level may be determined depending on factors including type, part, and severity of the wound, age, and sex of the patient, drug activity, drug sensitivity, dosing time, dosing route and excretion ratio, duration of treatment, and concurrently used medications, and other factors well-known in the medical field.

The pharmaceutical composition of this invention may contain a pharmaceutically acceptable carrier. The pharmaceutically acceptable carrier may be a conventional one for formulation, including lactose, dextrose, sucrose, sorbitol, mannitol, starch, rubber arable, potassium phosphate, arginate, gelatin, potassium silicate, microcrystalline cellulose, polyvinylpyrrolidone, cellulose, water, syrups, methyl cellulose, methylhydroxy benzoate, propylhydroxy benzoate, talc, magnesium stearate, and mineral oils, but is not limited thereto.

The pharmaceutical composition according to the present invention may further include a lubricant, a humectant, a sweetener, a flavoring agent, an emulsifier, a suspending agent, and a preservative. Details of suitable pharmaceutically acceptable carriers and formulations can be found in Remington's Pharmaceutical Sciences (19th ed., 1995), which is incorporated herein by reference.

The pharmaceutical composition of the present invention may be administered orally or parenterally, and examples of parenteral administration may include intraocular, intravitreal, intravenous, subcutaneous, intramuscular, intraperitoneal, and transdermal injections.

A suitable dose of the pharmaceutical composition of the present invention may vary depending on pharmaceutical formulation methods, administration methods, the patient's age, body weight, sex, severity of diseases, diet, administration time, administration route, an excretion rate and sensitivity for a used pharmaceutical composition. Preferably, the pharmaceutical composition of the present invention is administered with a daily dose of 0.0000001-100 mg/kg.

According to the conventional techniques known to those skilled in the art, the pharmaceutical composition may be formulated with pharmaceutically acceptable carrier and/or vehicle as described above, finally providing several forms including a unit dose form and a multi-dose form. Formulation may be oil or aqueous media, resuspension or emulsion, extract, powder, granule, tablet and capsule and further comprise dispersant or stabilizer.

According to an embodiment of the present invention, the composition of the present invention is an eye drop. The eye drop of the present invention may include a buffer, a preservative (including preservative adjurvant), an osmotic regulator, a surfactant, a solubilizer, a stabilizer, a softener (emollient), a pH adjuster, a malagma, and/or a lubricant, which are pharmaceutically acceptable.

### Advantageous Effects

Features and advantages of the present invention are summarized as follows.
(i) The present invention provides a pharmaceutical composition for reducing or inhibiting scar formation, the composition containing BMP-7 as an active ingredient, and hyaluronic acid, chitosan, and/or cyclodextrin as an excipient.
(ii) The composition of the present invention can be favorably used as an eye drop or the like for preventing the scar formation that may occur due to plastic surgery or laser surgery for the cornea.

### Brief Description of the Drawings

FIGS. 1a and 1b show effects of BMP-7 and an excipient on TGF-β-induced HaCaT cells. The cells were treated with TGF-β (5 ng/mℓ), BMP-7 (200 ng/mℓ), BMP-7 and hyaluronic acid (0.3% v/w), BMP-7 and chitosan (0.5% v/w), or BMP-7 and cyclodextrin (0.5% v/w), respectively, and incubated for 24 hours. Cell supernatant and extract were prepared, and the expressions of α-SMA and fibronectin were assayed by Western blot. [A] In order to verify the effect of BMP-7 according to the use in combination with excipient, intracellular or extracellular expressions of fibronectin and α-SMA were observed in HaCaT cells. [B] Fibronectin protein was quantified by ELISA. [C] The effect of BMP-7 or BMP-7 with excipient on the transcription of collagen type 1 mRNA was verified.
FIG. 2 shows immunofluorescence test results illustrating the effect of BMP-7 according to the use in combination with excipient in HaCaT cells. α-SMA (red) and fibronectin (green)
FIG. 3 shows the influence of BMP-7 with excipient on cell viability.
FIG. 4 shows results of comparing the effect of BMP-7 with the effect of TGF-β inhibitor in HaCaT cells.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### EXAMPLES

### Materials and Methods

### Cell culture

HaCaT cells were grown in Dulbeco's Modified Eagle's Medium (DMEM, Cell gro) supplemented with 10% fetal bovine serum (FBS, Cell gro), and 5% Penicillin-Streptomycin (Cell gro) in an atmosphere of 0.5% CO₂ at 37°C.

### Immunoblot analysis

Cells and supernatants were collected and lysed in RIPA buffer (Sigma) containing protease inhibitor (Sigma). The protein concentration was measured using Bradford assay system (Bio-Rad). Lysates were transferred to 1.5 ml eppendorf tubes and denatured by 5X sodium dodecyl sulfate (SDS) sample buffer (Invitrogen), and then boiled at 100°C for 5 minutes. 20 µg of proteins were loaded in 4∼12% SDS-PAGE gels (KOMA) and electrotransferred to NC or PVDF membrane (Bio-Rad, Invitrogen). After transfer, the membrane was incubated in blocking solution containing 5% skim milk in Tris-HCl buffered saline with 0.1% Tween 20 (TBST), for 1 hour. After 3 times washing with TSBT, the membrane was transferred to primary antibody solution and incubated overnight at 4°C. Anti-mouse α-SMA (Sigma) and anti-human fibronectin antibodies (Santa Cruz biotechnology) were used as primary antibodies and diluted in 5% of bovine serum albumin (BSA) in TBST. The membrane was washed in TBST three times for 5 minutes and was then incubated with secondary antibody for 1 hour at room temperature. HRP-conjugated goat anti-mouse IgG was used as secondary antibody and diluted in 5% skim milk in TBST. The membrane was washed in TSBT three times for 5 minutes and then immunocomplex was visualized with the ECL system (Santa Cruz biotechnology).

### ELISA

96-well plate was coated with 100 µl of anti-human fibronectin (Santa Cruz biotechnology, 1/100 diltion in PBS with 1% BSA) at 4°C for overnight. The plate was blocked with 200 µl PBS with 1% BSA at room temperature for 2 hours. Human plasma fibronectin (Millipore) and collected supernatant were inoculated at 37°C for 1.5 hours. The plate was washed with Phosphate Buffered Saline with Tween 20 (PBST) 5 times. Primary antibody (Rabbit anti-fibronectin antibodies, abcam) was incubated at room temperature for 2 hours. The plate was washed with PBST 5 times. Secondary antibody (anti-rabbit HRP-conjugated, Santa Cruz biotechnology) was incubated at room temperature for 1 hour. The plate was washed with PBST 5 times. Enzyme substrate (TMB, Invitrogen) was incubated at room temperature for 10 minutes. 50 µl of 2M H₂SO₄ was added to each well of the plate. Absorbance at 450nm was recorded using micro plate reader (Biotek).

### RT-PCR (Reverse transcription-Polymerase Chain Reaction)

Total cellular RNA were prepared with TRIzol (Invitrogen Life Technologies), according to the manufacturer's instructions. The following primers were used;
1. Collagen I: TCG GCG AGA GCA TGA CCG ATG GAT (sense, SEQ ID NO:1), GAC GCT GTA GGT GAA GCG GCT GTT, GAPDH (antisense, SEQ ID NO:2);
2. GAPDH: GCC AAA GGG TCA TCA TCT C (sense, SEQ ID NO:3), GTA GAG GCA GGG ATG ATG TT(antisense, SEQ ID NO:4).

First-strand cDNA was synthesized by ONE-STEP RT-PCR premix kit (Invitrogen) at 45°C for 30 minutes and 94°C for 5 minutes. PCR was performed using 500 ng of total RNA. After denaturation at 94°C for 30 seconds, 30 cycles were performed including annealing at 50°C for 30 seconds, extension at 72°C for 1 minute and final extension at 72°C for 5 minutes. Result fragments were identified by 3% agarose gel electrophoresis.

### Confocal microscopy

Cells at the density of 0.3 x 10⁵ were grown in a poly-L-lysine (Sigma) coated cover slip in 12 well tissue culture plates. Cells were washed with phosphate buffered saline (PBS) and fixed using 4% formaldehyde *in PBS for 15 minutes at room temperature. The cells were rinsed three times in PBS for 5 minutes and* permeabilized *with 100% ice-cold methanol for 10 minutes at -20°C. The plate was rinsed with PBS for 5 minutes and blocked with blocking buffer (5*% *BSA in PBS) for 1 hour at room temperature. Primary antibodies* (Anti-mouse α-SMA (Sigma) and anti-human fibronectin (Santa Cruz biotechnology)) *was applied in to the cells for overnight at 4°*C. *The cells were rinsed 3 times in PBS for 5 minutes. Secondary antibody* (Goat anti-mouse IgG-FITC, Goat anti-mouse IgG-TR, Santa Cruz biotechnology) diluted with *antibody dilution buffer (1% BSA in PBS) was incubated for 1 hour at room temperature.* After washing with PBS, the coverslips were incubated with DAPI. The cells was viewed by confocal lazer scanning microscope(Carl Zeiss). Image was captured using Program.

### Cell viability test

HaCat cells were prepared (2.5x10⁴, 1.25x10⁴, 6.2x10³, 3.1 x10³, or 1.6x10³) in a 96-well microplate in a final volume of 100 µl /well medium and incubated at 37□ for 48 hours. *Media is changed with serum-free DMEM media for 6 hours and incubated with each material (200* ng/ml BMP-7 with 0.3% v/w Hyaluronic acid (Sigma-Aldrich), 0.5% v/w Chitosan (Sigma-Aldrich) and 0.5% v/w Cyclodextrin (Sigma-Aldrich)) *for 24 hours. Cell viability test kit (its Bio) reagent was added to 10 µl per well to each well. Cells were incubated for 1 hour at 37°C. The plate was shook thoroughly for 1 minute on a shaker. The absorbance of the samples was measured using a microtiter plate reader (Biotek) at 450 nm.*

### Results

### Effect of BMP-7 with excipients

The expression of intracellular or extracellular fibronectin and α-SMA were observed in HaCaT cell to verify BMP-7 efficacy with excipients. As shown in A of Fig. 1a, expression of fibronectin and α-SMA upregulated by TGF-β induction (lane2), but treatment of BMP-7 significantly reduced the expression of fibronectin and α-SMA (lane 4). Similarly, the expression of these proteins was reduced by BMP-7 with different excipients (lane 5, 6, 7).

The fibronectin protein was quantified by ELISA. As a result, as shown in B of FIG. 1a, the expression levels of fibronectin were reduced by the combined treatment of BMP-7 and expient.

The effects of BMP-7 and a combination of BMP-7 and excipient on collagen type 1 mRNA transcription were verified. As a result of RT-PCR, as shown in C of FIG. 1b, the expression level of collagen transcript was upregulated by TGF-β, but the expression level of the transcript was reduced by BMP-7 or a combined treatment of BMP-7 and excipient. These results indicate that the combination of BMP-7 and excipient can inhibit fibrosis of HaCaT cells.

### Immunofluorescence test results

The expressions of α-SMA and fibronectin in HaCaT cells were verified using an immunofluorescence method and a cofocal microscope. As a result of verification, as shown in FIG. 2, α-SMA (red) or fibronectin (green) was expressed by TGF-β in HaCaT cells, but the expressions of the two proteins were reduced by BMP-7 or a combined treatment of BMP-7 and excipient.

### Verification on effect of BMP-7 with excipient on cell viability

In order to verify the applicability to an eye drop, the cytotoxicity of BMP-7 and/or excipient was verified. As a result, as shown in FIG. 3, BMP-7 and excipient had no significant effect on cell viability compared with the control group. These results indicate that hyaluronic acid, chitosan, and cyclodextrin do not exhibit toxicity on cells, and thus can be safely used as excipients together with BMP-7.

### Comparison between BMP-7 and TGF-β inhibitor

Cells were treated with TGF-β (5 ng/mP) alone, BMP-7 (200 ng/mℓ) alone, TGF-β and BMP-7, TGF-β and heparin (100 *µ*g/mℓ), or TGF-β and TGF-β inhibitor ((+) Control of p38 inhibitor (50 µM)), and then incubated for 24 hours. Cell extract was prepared, and the expression of fibronectin was assayed by Western blot. In addition, the fibronectin protein was quantified by ELISA. That is, in order to compare the effect between BMP-7 and heparin, the extracellular (HaCaT) fibronectin expression was measured.

As a result, as shown in FIG. 4, the expression of fibronectin was induced by TGF-β, but BMP-7 effectively inhibited the expression of TGF-β-induced fibronectin, like heparin.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A pharmaceutical composition for reducing or inhibiting scar formation, the composition containing:
(a) a pharmaceutically effective amount of bone morphogenetic protein 7 (BMP-7); and
(b) at least one excipient selected from the group consisting of hyaluronic acid, chitosan, and cyclodextrin.

2. The composition of claim 1, wherein the scar is formed by the healing procedure of a wound on the surface of an eyeball.

3. The composition of claim 1, wherein the composition is an eye drop.

4. A method for reducing or inhibiting scar formation, the method comprising administering to a subject, a therapeutically effective amount of a pharmaceutical composition containing at least one excipient selected from the group consisting of hyaluronic acid, chitosan, and cyclodextrin.

5. The composition of claim 4, wherein the composition is a composition for administration to an eyeball surface.
